# EUROPEAN PATENT APPLICATION

(11) **EP 4 151 238 A1**
(43) Date of publication of application: **22.03.2023**
(21) Application number: 22808564.3
(22) Date of filing: 05.07.2022
(51) Int. Cl.: A61K 49/00, A61K 41/00, A61K 47/02, A61K 47/04, A61P 35/00

(54) **PHARMACEUTICAL PREPARATION FOR LOW-TEMPERATURE THERMOTHERAPY, AND PREPARATION METHOD THEREFOR AND USE THEREOF**

(30) Priority: 13.07.2021 CN 202110791901
(71) Applicant: Sichuan Enray Pharmaceutical Technology Co., Ltd., Chengdu, Sichuan 610041 (CN)
(72) Inventor: TANG, Xiaohai, Chengdu, Sichuan 610041 (CN); TANG, Kexin, Chengdu, Sichuan 610041 (CN); HUANG, Yuanfang, Chengdu, Sichuan 610041 (CN)
(74) Representative: Huang, Liwei
(86) International application number: PCT/CN2022/103774
(87) International publication number: WO 2023/284582

(57) **Abstract**

The present invention claims an application of a CNSI-Fe compound preparation in preparing a tumor hyperthermia drug used in conjunction with near-infrared light. When the compound preparation is used for low-temperature hyperthermia, the administration dosage can be decreased so as to reduce the occurrence of adverse reactions; the hyperthermia using the compound preparation has a low temperature, so that the surrounding normal tissues are not liable to be damaged, and the adverse reactions during the hyperthermia using carbon nanoparticles can also be reduced. Moreover, it is further proved that the CNSI-Fe compound preparation and the near-infrared irradiation are synergic. Based on the means and technical effects of low-temperature hyperthermia, the present invention has extensive clinical significance.

## Description

### TECHNICAL FIELD

The present invention belongs to the technical field of medicines, and particularly relates to a pharmaceutical preparation for low-temperature hyperthermia as well as a preparation method and an application thereof.

### BACKGROUND

Photothermal therapy is a new tumor therapy developed recently, and it has great application prospects because of its small trauma, obvious therapeutic effect and lower toxic and side effects. In the development process of reagents for hyperthermia, carbon nanotubes, graphene, carbon quantum dots and fullerene are the hot directions in the current researches. The carbon atoms in these materials form covalent bonds by SP2 hybridization and extend around to form a foveolate six-membered ring structure, π electrons are enriched on the ring to form delocalized π bonds. Therefore, the SP2 hybridization gives these carbon materials many special properties, including absorbing NIR light. After absorbing NIR, these carbon materials can quickly convert light energy into heat to kill tumor cells, so that they are ideal potential candidate materials for hyperthermia.

After long-term accumulation of scientific research data, great progress has been obtained in the development of reagents for hyperthermia, including researches on the effective improvement of heat conversion efficiency, the surface modification for improving targeting ability and toxicity. However, there are also many problems limiting the clinical application in the research process. The main defects are as follows: (1) The preparation process is difficult to control. The crude products of carbon nanotubes, graphene and fullerene contain a lot of impurities, and toxic and side effects may be induced in vivo; the poor hydrophilicity is not conducive to dispersion in water, and thus refining and surface modification are required. In the refining process, impurities are mainly removed to improve purity and reduce the risk of toxic and side effects caused by impurities. In the surface modification, surface groups of carbon are mainly modified, which is conducive to the dispersion in water and improves the conversion efficiency of NIR during hyperthermia. However, it is found in the process of reagent development that these carbon materials are mixtures with a certain range of particle sizes, and it is difficult to control the consistency between batches in the refining and surface modification process, thereby leading to differences in product properties between batches, affecting the comprehensive evaluation, and hindering the implementation of later tests. (2) The shape and size of particles are difficult to control. In these materials, the carbon nanotubes are in a tubular structure, both ends need to be passivated during the preparation, but some needle-tip-like ends still exist usually due to incomplete treatment; after administration, these sharp ends tend to pierce blood vessel walls and then enter nucleuses, so that there is potential genetic toxicity. (3) The targeting ability is poor and the accuracy is insufficient. After local administration, owing to poor targeting ability, the carbon nanotubes, graphene, carbon quantum dots and fullerene are enriched around tumors, and some of them will diffuse to normal tissues. In the hyperthermia, the carbon diffused around the normal tissues will also become hot to result in burns to the normal tissues. (4) The cost is high. Because the complex preparation process involves the steps of preparation, refining and chemical modification, the cost is too high to limit its large-scale production and clinical applications. Under the above-mentioned application background, the application of preparations for hyperthermia has been limited to an early exploration stage, and there is no safe, stable and controllable reagent entering at a clinical application stage to be safely and effectively used by patients.

In addition, the heating principle of carbon nanoparticles is that carbon nanoparticles can efficiently absorb near-infrared ray (NIR) and convert NIR into heat, but body tissues have weak absorption of NIR (high transmittance), and less NIR is converted into heat. Therefore, taking advantage of the above differences, carbon nanoparticles are used as a medium of hyperthermia; after carbon nanoparticles are injected into a cancer focus, the externally irradiated NIR can reach the cancer focus through a surface normal tissue and heat the carbon nanoparticles, thereby raising the temperature of the tumor tissue to produce an effect of hyperthermia. However, the hyperthermia using carbon nanoparticles requires a temperature as high as 50°C or above to result in necrosis and coagulation of tumor cells, and the higher temperature may easily damage cells of adjacent normal tissues. Carbon nanoparticles suspension injection-Fe, a previously developed drug, is a nanosuspension taking carbon nanoparticles as a carrier and ferrous ions as an effective component, and is an innovative anti-cancer drug. Its mechanism of action is as follows: after being locally injected into a cancer tissue, the carbon nanoparticles suspension injection-Fe (CNSI-Fe) enters cancer cells through over-expressed iron channels on cancer cell membranes. A large number of iron ions enter the cancer cells rich in hydrogen peroxide (H2O2) and then have a Fenton reaction with the H2O2 to produce a plenty of hydroxyl radicals (·OH); the ·OH with extremely strong oxidation properties reacts with unsaturated fatty acids (UPFAs) in the cells to produce a large number of highly destructive lipid hydrogen peroxide (L-OOH), that is, lipid reactive oxygen species (lipid-ROS), and the lipid-ROS can destroy organelles to lead to cell damage, thereby resulting in ferroptosis.

CNSI-Fe2+ + H2O2 →ROS ( ·OH) →L-ROS→Ferroptosis

The influencing factors of the Fenton reaction include a pH value, an H2O2 dosage, an Fe2+ dosage, a reaction time and a reaction temperature. When the other conditions remain unchanged, increasing the reaction temperature can speed up the reaction and enhance the reaction rate. After CNSI-Fe is locally injected into a cancer tissue, near-infrared irradiation can elevate its temperature to enhance the Fenton reaction of iron ions. However, at present, the temperature of hyperthermia directly using a carbon nanoparticles preparation in the prior art is usually higher than 50°C, and the surrounding skin will often be burned if the temperature is higher than 50°C. Therefore, there is an urgent need in the field for a preparation that can give full play to carbon nanoparticles as a drug preparation for hyperthermia, without burning the skin and with convenience for patients to use.

### SUMMARY

In order to overcome the above-mentioned technical defects, the present invention provides a new application of a CNSI-Fe, and the following technical solutions are specifically adopted:
The first aspect of the present invention provides an application of a CNSI-Fe compound preparation in preparing a tumor hyperthermia drug used in combination with near-infrared light.

Further, the "used in combination with near-infrared light" refers to heating a tissue using near-infrared light; still further, a wavelength of the near-infrared light is 780-2,600 nm; and still further preferably, the wavelength of the near-infrared light is 780-1,400 nm.

Further, the CNSI-Fe compound preparation includes a carbon nanoparticles suspension injection (CNSI) and ferrous sulfate for injection, wherein a mass ratio of the CNSI to the ferrous sulfate for injection is 20-100:3-300; preferably, the mass ratio is 50:7.5-180; and more preferably, the mass ratio is 50:15-45.

Further, when the CNSI-Fe compound preparation is used, the CNSI and the ferrous sulfate for injection are uniformly mixed according to the above-mentioned mass ratio, and a pH value of the mixture is determined to be 2-7; preferably, the pH value is determined to be 3-5.5.

Further, the CNSI-Fe includes carbon nanoparticles, a suspending agent, saline, a pH adjuster, sodium chloride and water for injection; still further, each 1,000 mL of CNSI includes 20-100 g of carbon nanoparticles, 17-30 g of suspending agent, 2-4 g of pH adjuster, 8-10 g of sodium chloride and a remaining amount of water for injection; and more preferably, each 1,000 mL of CNSI includes 50 g of carbon nanoparticles, 20 g of suspending agent, 3 g of pH adjuster, 9 g of sodium chloride and a remaining amount of water for injection.

Still further, the carbon nanoparticles are any one or more selected from carbon nanoparticles, carbon nanotubes, carbon quantum dots, graphene, fullerenes, carbon nanorods and carbon nanofibers; preferably, the carbon nanoparticles are carbon nanoparticles or graphene; and more preferably, the carbon nanoparticles are carbon black.

Still further, the ferric salt is any one or more selected from ferrous sulfate, ferric sulfate, ferrous chloride, ferric trichloride, ferrous gluconate, ferric sucrose, ferric ammonium citrate, ferrous succinate, ferric sorbitol and ferrous fumarate; preferably, the ferric salt is ferrous sulfate, ferric sulfate, ferrous chloride or ferric chloride; and more preferably, the ferric salt is ferrous sulfate.

Still further, the suspending agent is any one or more selected from poloxamer, polyvinylpyrrolidone C30 (PVPC30) and Tween-80; and preferably, the suspending agent is poloxamer.

Still further, the pH adjuster is any one selected from sodium citrate, sodium acetate, sodium borate, sodium phosphate and sodium bicarbonate; preferably the pH adjuster is sodium citrate, and the sodium citrate forms a complex compound with ferrous and/or ferric ions in the ferric salts.

Still further, a preparation method of the CNSI includes the following steps:
1) taking 90% v/v of the formulation amount of water for injection, adding the formulation amount of sodium chloride, and stirring the mixture until the mixture is completely dissolved to obtain a salt solution;
2) adding the formulation amounts of pH adjuster and suspending agent into the salt solution obtained in step 1), and stirring the mixture until the mixture is completely dissolved to obtain an excipient solution;
3) adding the formulation amount of pretreated carbon nanoparticles into the excipient solution obtained in step 2), stirring the mixture evenly, and then adding the remaining amount of water for injection to a constant volume to obtain a constant volume solution; and
4) homogenizing the constant volume solution obtained in step 3) at a rate of 15,000-20,000 rpm for 3-10 min and then at a pressure of 15,000-30,000 psi for 1-5 times, then performing filling and capping, and finally performing steam sterilization at 115-130°C for 10-30 min to obtain the CNSI.

Further, the pretreatment in step 3) includes the following steps: firstly washing the carbon nanoparticles degreased by ethyl acetate using a 8-15%v/v HNO3 aqueous solution, wherein a mass-to-volume ratio of the carbon nanoparticles to the HNO3 aqueous solution is 1 g : 3-5/ml; then washing the carbon nanoparticles with water to be approximately neutral; then washing the carbon nanoparticles with a 0.08-0.15 mol/L NaOH aqueous solution, wherein a mass-to-volume ratio of the carbon nanoparticles to the NaOH aqueous solution is 1 g : 3-5/ml; and washing the carbon nanoparticles with water to be approximately neutral; still further, the mass-to-volume ratio of the carbon nanoparticles to the HNO3 aqueous solution in S1 is 1 g:4/ml; and preferably, the HNO3 is a 10%v/v HNO3 aqueous solution.

Further, in step 4), the homogenization rate is 18,000 rpm, and the homogenization time is 5 min.

Further, in step 4), the homogenization pressure is 20,000 psi, and the homogenization is performed for 3 times.

Further, in step 4), the sterilization temperature is 121°C, and the sterilization time is 115 min.

Further, the ferrous sulfate for injection is a lyophilized powder of ferrous sulfate for injection.

Still further, the lyophilized powder of ferrous sulfate for injection is prepared from the following raw materials: ferrous sulfate heptahydrate, sulfuric acid and water for injection, wherein a mass ratio of ferrous sulfate heptahydrate : sulfuric acid : water for injection is 0.1-0.2 g : 0.4-0.85 µg : 2 g; and preferably, the sulfuric acid is 1% (by weight) sulfuric acid.

Still further, a preparation method of the lyophilized powder of ferrous sulfate for injection includes the following steps:
S1: taking 90% of the formulation amount of water for injection, and continuously filling N2 below the liquid level during the preparation, with a nitrogen flow rate controlled at 4.0-5.0 m³/h;
S2: then adding sulfuric acid to adjust the pH value to 2.4-2.8; and preferably, the pH value is adjusted to 2.4;
S3: taking the formulation amount of ferrous sulfate heptahydrate, and fully stirring the ferrous sulfate heptahydrate for dissolution;
S4: adding the remaining amount of sulfuric acid to adjust the pH value to 2.8-3.0, adding the remaining amount of purified water to fix the volume to a full volume, and stirring the mixture evenly to obtain a ferrous sulfate solution; and preferably, the pH value is adjusted to 2.8;
S5: filtering the obtained solution using a microporous filter membrane, then performing filling, lyophilization, vacuum plugging and capping; and preferably, a pore diameter of the microporous filter membrane is 0.45 µm.

Further, the lyophilization step in S5 includes the following specific operations:
S51 pre-freezing: rapidly lowering the temperature to (-10 to -5)°C and keeping the temperature for 1-3 h, and then rapidly lowering the temperature to (-50 to -40)°C and keeping the temperature for 1-3 h; preferably, the temperature is lowered to -5°C and kept for 2 h; and preferably, the temperature is lowered to -45°C and kept for 2 h;
S52 drying: S521: gradually elevating the temperature from (-50 to -40)°C to (-20 to -10)°C within 2-4 h, and keeping the temperature at (-20 to -10)°C and 80-120 mtorr for 2-4 h; preferably, the temperature is gradually elevated from -45°C to -15°C within 3 h and kept at -15°C and 100 mtorr for 3 h;
S522: gradually elevating the temperature from (-20 to -10)°C to (-10 to -0)°C within 1-3 h, and keeping the temperature at 80-120 mtorr and (-10 to -0)°C for 1-3 h; preferably, the temperature is gradually elevated from -15°C to -5°C within 2 h and kept at 100 mtorr and 100 -5°C for 2 h; and
S523: gradually elevating the temperature from (0-20)°C within 3-6 h, and keeping the temperature at 80-120 mtorr and 20°C for 3-6 h; and preferably, the temperature is gradually elevated from 0°C to 20°C within 4 h and kept at 100 mtorr and 20°C for 4 h.

### Beneficial effects

The present invention discloses a CNSI-Fe compound preparation which can be used for the low-temperature hyperthermia of tumor. Compared with carbon nanoparticles preparations, when the pharmaceutical composition is used for low-temperature hyperthermia, the administration dosage can be significantly decreased so as to reduce the occurrence of adverse reactions; the hyperthermia using the compound preparation of the present invention has a lower temperature, so that the surrounding normal tissues are not liable to be damaged, and the adverse reactions during the hyperthermia using carbon nanoparticles can also be reduced. Moreover, it is further proved that CNSI-Fe compound preparation and the near-infrared irradiation are synergic. Based on the means and technical effects of low-temperature hyperthermia, the present invention has extensive clinical significance.

### BRIEF DESCRIPTION OF THE DRAWINGS

FIG. 1 shows growth curves of gross tumor volumes of xenograft tumor of SMMC-7721 liver cancer nude mice treated in all groups.
FIG. 2 shows growth curves of gross tumor volumes of xenograft tumor of HCT-116 colon cancer nude mice treated in all groups.
FIG. 3 shows growth curves of gross tumor volumes of xenograft tumor of MDA-MB-231 breast cancer nude mice treated in all groups.
FIG. 4 is an oscillogram of hydroxyl radicals in tumor tissues of SMMC-7721 liver cancer.
FIG. 5 shows inhibitory effects on lymph node metastasis of H22 liver cancer cells in all groups.

### DETAILED DESCRIPTION OF THE EMBODIMENTS

### (I) Examples

### Example 1: preparation of CNSI

For a CNSI, each 1,000 mL of CNSI included 50 g of carbon nanoparticles, 20 g of suspending agent, 3 g of pH adjuster, 9 g of sodium chloride and a remaining amount of water for injection; and a preparation method included the following steps:
1) 90% v/v of the formulation amount of water for injection was taken, the formulation amount of sodium chloride was added, and the mixture was stirred until the mixture was completely dissolved to obtain a salt solution; 2) the formulation amounts of pH adjuster and suspending agent were added to the salt solution obtained in step 1), and the mixture was stirred until the mixture was completely dissolved to obtain an excipient solution; 3) the formulation amount of pretreated carbon nanoparticles was added to the excipient solution obtained in step 2), the mixture was stirred evenly, and then the remaining amount of water for injection was added to a constant volume to obtain a constant volume solution, wherein the pretreatment included the following steps: firstly the carbon nanoparticles degreased by ethyl acetate was washed using a 10%v/v HNO3 aqueous solution, wherein a mass-to-volume ratio of the carbon nanoparticles to the HNO3 aqueous solution was 1 g : 4/ml, and then the carbon nanoparticles were washed with water to be approximately neutral; then the carbon nanoparticles were washed with a 0.10 mol/L NaOH aqueous solution, wherein a mass-to-volume ratio of the carbon nanoparticles to the NaOH aqueous solution was 1 g : 4/ml, and then the carbon nanoparticles were washed with water to be approximately neutral; and 4) the constant volume solution obtained in step 3) was homogenized at a rate of 18,000 rpm for 5 min and then at a pressure of 20,000 psi 3 times, then filled and capped, and finally sterilized by steam at 121°C for 15 min to obtain the CNSI.

### Example 2: preparation of ferrous sulfate for injection

A lyophilized powder of ferrous sulfate for injection was prepared from the following raw materials: ferrous sulfate heptahydrate, sulfuric acid and water for injection, wherein a mass ratio of ferrous sulfate heptahydrate : sulfuric acid : water for injection was 0.149 g : 0.4-0.85 µg : 2 g; preferably, the sulfuric acid was 1% (by weight) sulfuric acid. A preparation method included the following steps:
S1: 90% of the formulation amount of water for injection was taken, N2 was continuously filled below the liquid level during the preparation, and a nitrogen flow rate was controlled at 4.0-5.0 m³/h; S2: sulfuric acid was then added to adjust the pH value to 2.4; S3: the formulation amount of ferrous sulfate heptahydrate was taken and fully stirred for dissolution; S4: the remaining amount of sulfuric acid was added to adjust the pH value to 2.8, the remaining amount of purified water was added to fix the volume to a full volume, and the mixture was stirred evenly to obtain a ferrous sulfate solution; and S5: the obtained solution was filtered with a 0.45 µm microporous filter membrane, filled, lyophilized, vacuum plugged and capped to obtain the lyophilized powder; wherein the lyophilization step included the following specific operations:
S51 pre-freezing: the temperature was rapidly lowered to -5°C and kept for 2 h, and then the temperature was rapidly lowered to -45°C and kept for 2 h; S52 drying: S521: the temperature was gradually elevated from -45°C to -15°C within 3 h and kept at -15°C and 100 mtorr for 3 h; S522: the temperature was gradually elevated to -15 to -5°C within 2 h and kept at -5°C and 100 mtorr for 2 h; and S523: the temperature was gradually elevated to 0-20°C within 4 h and kept at 20°C and 100 mtorr for 4 h.

In examples 3-22, the CNSI and the ferrous sulfate for injection in different proportions and near-infrared light of different wavelengths were selected for hyperthermia of tumor.

| | **CNSI** | **Ferrous Sulfate for Injection** | **Infrared Wavelength (nm)** |
|---|---|---|---|
| **Example 3** | 50 mg | 21 mg | 780 |
| **Example 4** | 50 mg | 21 mg | 808 |
| **Example 5** | 50 mg | 21 mg | 1,064 |
| **Example 6** | 50 mg | 21 mg | 1,400 |
| **Example 7** | 50 mg | 21 mg | 2,600 |
| **Example 8** | 50 mg | 15 mg | 1,064 |
| **Example 9** | 50 mg | 45 mg | 808 |
| **Example 10** | 50 mg | 7.5 mg | 808 |
| **Example 11** | 50 mg | 90 mg | 808 |
| **Example 12** | 50 mg | 180 mg | 808 |
| **Example 13** | 50 mg | 3 mg | 808 |
| **Example 14** | 50 mg | 300 mg | 808 |
| **Example 15** | 20 mg | 3 mg | 808 |
| **Example 16** | 20 mg | 30 mg | 808 |
| **Example 17** | 20 mg | 150 mg | 808 |
| **Example 18** | 20 mg | 300 mg | 808 |
| **Example 19** | 100 mg | 3 mg | 808 |
| **Example 20** | 100 mg | 30 mg | 808 |
| **Example 21** | 100 mg | 150 mg | 808 |
| **Example 22** | 100 mg | 300 mg | 808 |

### (II) Experimental examples

### 1. Experimental materials:

### 1) Cell strains

SMMC7721 liver cancer cells, HCT116 colon cancer cells, MDA-MB-231 breast cancer cells, and mouse derived liver cancer H22 cells

### 2) Cell culture media

DMEM cell medium, RMPI1640 medium, fetal bovine serum (FBS), trypsin for cell digestive solution, penicillin-streptomycin mixture, and phosphate buffer solution (PBS, pH 7.4)

### 3) Experimental animals

BalB/c-nu mice, male, 5-7 weeks old, and weighing 20±2 g. The mice could drink and eat freely during the experiments. The mice were irradiated by light for 12 h every day, and fed in isolated cages with separate air supply, with 5 mice in each cage.

SPF-class Balb/c mice, male, 4-6 weeks old, and weighing 20±2 g. The mice could drink and eat freely during the experiments. The mice were irradiated by light for 12 h every day, and fed in isolated cages with separate air supply, with 5 mice in each cage.

### 4) Experimental drugs and main instruments

CNSI-Fe for example 4, CNSI-Fe for example 8, CNSI (with a concentration the same as those used in examples 4 and 8), 0.9% sodium chloride injection, blast drying oven, thermostatic water bath kettle, biological optical microscope, thermostatic incubator, water purifier, autoclave, ultra-clean workbench, electronic balance, 808nm near-infrared hyperthermia instrument, 1,064nm laser hyperthermia instrument, infrared thermograph, and electron spin resonance (ESR) spectrometer.

### 2. Experimental methods

### (1) Cell experiment

Cells in a logarithmic growth phase were collected and counted, a cell density was adjusted to 30,000 cells/ml, and 1 ml was added to a 25×25 mm glass dish and incubated with 5% CO2 at 37°C for 24 h. The cells were divided into a negative group, a near-infrared irradiation group, a CNSI-Fe group, a CNSI + near-infrared irradiation group (37°C, 42°C, 45°C, 48°C and 50°C), and a CNSI-Fe + near-infrared irradiation group (37°C, 42°C, 45°C, 48°C and 50°C), with 3 duplicates for each group. The negative group and the near-infrared irradiation group were replaced with new culture solutions, and the CNSI + near-infrared irradiation group and the CNSI-Fe + near-infrared irradiation group were replaced with culture solutions containing CNSI or CNSI-Fe. The CNSI + near-infrared irradiation group and the CNSI-Fe + near-infrared irradiation group were irradiated to the required temperatures using the 808nm near-infrared hyperthermia instrument, respectively, and the temperatures were maintained for 10 min. The irradiation time of the near-infrared radiation group was consistent with the longest irradiation time of the test group. After irradiation, the cells were cultured for another 48 h. The cells were digested with trypsin and counted, and a cell inhibition rate was calculated.

### 2) Experiment on inhibiting tumor growth

Cells in a logarithmic growth phase were collected, a concentration of the cell suspension was adjusted to 3×107 cells/mL, and the cells were inoculated subcutaneously in the upper right limb of each nude mouse at 0.1 mL/mouse (including about 3×106 cells); when an average tumor volume of the inoculated mice reached 100 mm3, and the tumor bearing mice were randomly divided into a negative control group, a CNSI-Fe group, a CNSI + near-infrared irradiation group and a CNSI-Fe + near-infrared irradiation group (adopting the CNSI-Fe used in example 4). The method of administration was intratumoral injection, and a volume of administration was 50 µL/time; the second administration was given after an interval of 2 days, and the mice were administrated twice in total. At 10 min after the intratumoral injection, tumors were irradiated by 808 nm near-infrared ray in the CNSI + near-infrared irradiation group and the CNSI-Fe + near-infrared irradiation group, respectively, wherein a power density was 0.5 W/cm2, an irradiation time was 30 min, and a temperature was maintained at about 45°C. The near-infrared radiation group was irradiated for the same period of time. The tumor volume of the mice were measured every week, and the volume was calculated by the following formula: volume=(length × width2)/2. The observation lasted for 21 days.

At 24 h after the near-infrared irradiation, the tumor tissue was taken from each mouse, 0.9% sodium chloride injection was added to prepare a 10% homogenate, a capture agent was added, and hydroxyl radicals were detected with ESR.

### 3) Experiment on inhibiting lymph node metastasis

Milky white ascite was extracted from each tumor bearing mouse with H22 liver cancer and added to normal saline, and the mixture was centrifugated and re-suspended; the number of cells was adjusted to 3×107 cells/mL, the cells were inoculated subcutaneously on the foot pad of the left hind limb of each Balb/c mouse, and each mouse was inoculated with 50 µL to obtain a mouse model of cancer lymph node metastasis. The mice were treated when a tumor diameter reached 6-8 mm and there was neither ulcer nor necrosis. The mice were randomly divided into 4 groups, with 7 mice in each group, that is, a negative control group, a CNSI-Fe group, a near-infrared irradiation group, a CNSI + near-infrared irradiation group and a CNSI-Fe + near-infrared irradiation group (adopting the CNSI-Fe used in example 8). The method of administration was intratumoral injection, and a volume of administration was 50 µL/time; the second administration was given after an interval of 2 days, and the mice were administrated twice in total. At 10 min after the intratumoral injection of drugs, each mouse was irradiated in the position of axillary lymph nodes in the left hind limb by 1,064 nm near-infrared ray in the CNSI + near-infrared irradiation group and the CNSI-Fe + near-infrared irradiation group, respectively, wherein a power density was 0.5 W/cm2, an irradiation time was 30 min, and a temperature was maintained at about 45°C. The near-infrared radiation group was irradiated for the same period of time. At 2 weeks after the irradiation, the mice were killed, and the axillary lymph nodes were collected and weighed.

### 3. Experimental results:

### (1) Cell experiment

In the near-infrared irradiation group, there was no obvious killing effect on the SMMC7721 liver cancer cells, the HCT116 colon cancer cells and the MDA-MB-231 breast cancer cells, and the inhibition rates were all less than 10%. The inhibition rates of the CNSI-Fe acting alone to the SMMC7721 liver cancer cells, the HCT116 colon cancer cells and the MDA-MB-231 breast cancer cells were 40.15±2.98%, 34.97±1.67% and 32.85±3.07%, respectively. The inhibition rates to the three kinds of cells in the CNSI + near-infrared irradiation group and the CNSI-Fe + near-infrared irradiation group are as shown in Tables 1-3. When the temperature was 37°C and 40°C, there was basically no effect on the cells, and the inhibition rates gradually increased as the temperature was elevated. When the temperature was ≥ 42°C, the inhibition rate in the CNSI-Fe + near-infrared irradiation group was greatly increased and was better than those in the CNSI-Fe group and the CNSI + near-infrared irradiation group at the same temperature. In the CNSI + near-infrared irradiation group, when the temperature reached 50°C, the inhibition rate to the three kinds of cells reached above 90%; but in the CNSI-Fe + near-infrared irradiation group, the inhibition rate to the three kinds of cells reached above 90% when the temperature was 45°C.

**Table 1 Inhibition Rates to SMMC7721 Liver Cancer Cells in the CNSI + Near-infrared Irradiation Group and the CNSI-Fe + Near-infrared Irradiation Group**

| Temperature | Inhibition Rate (%) | |
|---|---|---|
| | CNSI + Near-infrared Irradiation Group | CNSI-Fe + Near-infrared Irradiation Group |
| 37°C | 4.92±2.94 | 38.95±3.68 |
| 40°C | 6.79±1.57 | 41.92±2.06 |
| 42°C | 39.03± 4.11 | 70.31± 1.53 |
| 45°C | 57.18± 2.67 | 92.77±1.14 |
| 48°C | 76.18± 3.41 | 98.46±2.64 |
| 50°C | 91.14±1.90 | 99.58±2.47 |

**Table 2 Inhibition Rates to HCT116 Colon Cancer Cells in the CNSI + Near-infrared Irradiation Group and the CNSI-Fe + Near-infrared Irradiation Group**

| Temperature | Inhibition Rate (%) | |
|---|---|---|
| | CNSI + Near-infrared Irradiation Group | CNSI-Fe + Near-infrared Irradiation Group |
| 37°C | 6.49±3.75 | 35.89±4.12 |
| 40°C | 10.98±2.85 | 37.15±3.48 |
| 42°C | 32.44±1.72 | 65.78±2.45 |
| 45°C | 60.54±4.82 | 90.64±3.56 |
| 48°C | 70.15±3.47 | 97.22±1.68 |
| 50°C | 92.86±3.86 | 99.69±2.76 |

**Table 3 Inhibition Rates to MDA-MB-231 Breast Cancer Cells in the CNSI + Near-infrared Irradiation Group and the CNSI-Fe + Near-infrared Irradiation Group**

| Temperature | Inhibition Rate (%) | |
|---|---|---|
| | CNSI + Near-infrared Irradiation Group | CNSI-Fe + Near-infrared Irradiation Group |
| 37°C | 8.10±4.45 | 37.66±3.28 |
| 40°C | 15.46±2.75 | 45.52±2.79 |
| 42°C | 42.38±3.27 | 73.91±1.05 |
| 45°C | 55.43±1.41 | 90.55±3.72 |
| 48°C | 75.25±2.72 | 98.47±3.61 |
| 50°C | 93.75±2.76 | 99.74±4.04 |

### 2) Experiment on inhibiting tumor growth

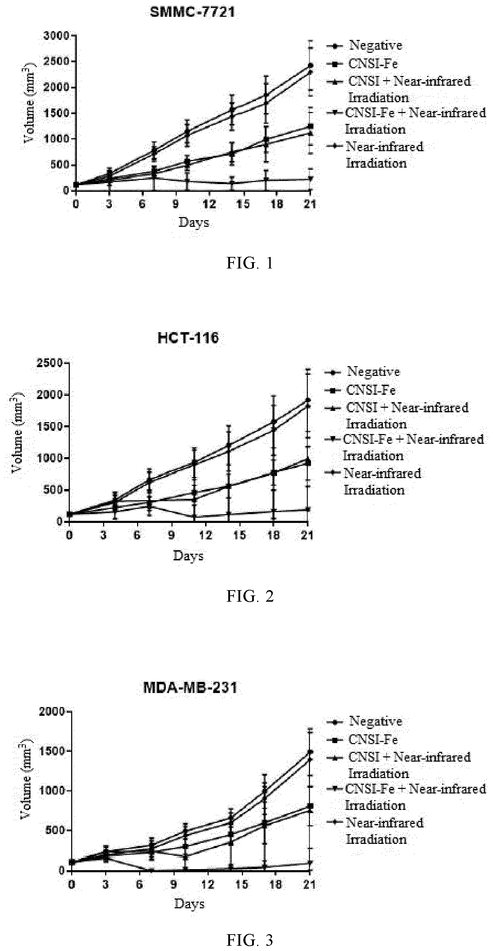

The inhibitory effects of CNSI-Fe, CNSI + near-infrared irradiation, CNSI-Fe + near-infrared irradiation, and near-infrared irradiation on the growth of subcutaneous xenograft tumor of the nude mice with SMMC7721 liver cancer cells, HCT116 colon cancer cells and MDA-MB-231 breast cancer cells were observed (FIGS. 1-3). The results showed that at the end of the observation, the near-infrared irradiation alone had no obvious inhibitory effect on the subcutaneous xenograft tumor of the three kinds of cells. The inhibition rates of the CNSI-Fe to the subcutaneous xenograft tumor of the three kinds of cells were 48.57%, 52.03% and 45.79%, respectively; the inhibition rates of the CNSI + near-infrared irradiation to the subcutaneous xenograft tumor of the three kinds of cells were 53.81%, 48.06% and 49.35%, respectively; and the inhibition rates of the CNSI-Fe + near-infrared irradiation to the subcutaneous xenograft tumor of the three kinds of cells were 90.78%, 90.14% and 93.3%, respectively. Comparing the CNSI-Fe + near-infrared irradiation with the CNSI-Fe alone or the CNSI + near-infrared irradiation, there were significant differences. According to the calculation using the Jin's formula method, the q value of the CNSI + near-infrared irradiation was greater than 1, indicating that CNSI-Fe and near-infrared irradiation were synergic and could enhance the anti-tumor effect of hyperthermia using CNSI-Fe and CNSI.

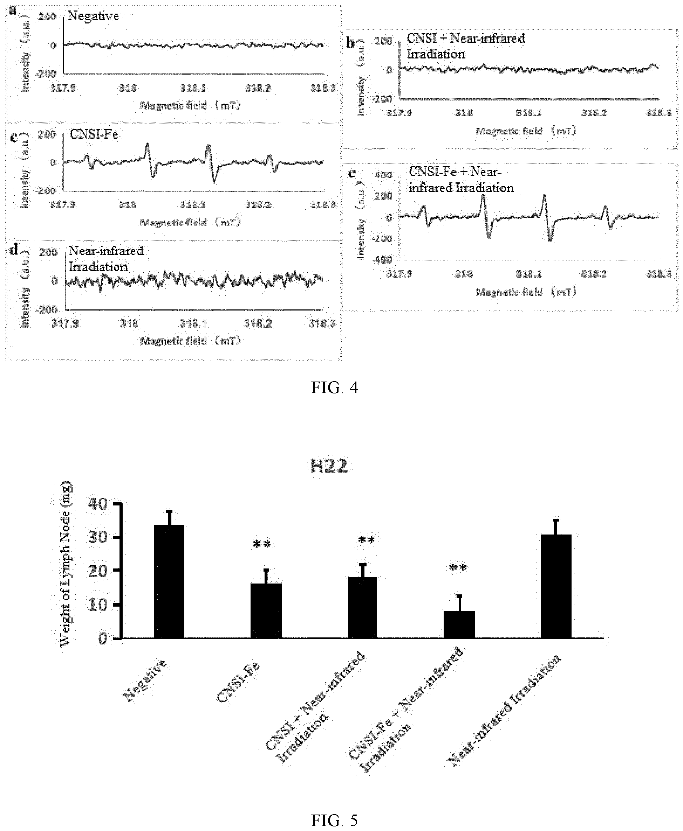

At the same time, the content of hydroxyl radicals in tumors of animals was compared among the groups (FIG. 4). The results respectively showed that FIG. 4a represented that the samples were not treated by any method; FIG. 4b and FIG. 4d represented that there were not significant hydroxyl radicals produced by the CNSI + near-infrared irradiation and the near-infrared irradiation, and the CNSI-Fe group (FIG. 4c) and the CNSI-Fe + near-infrared irradiation group (FIG. 4e) had the highest contents of hydroxyl radicals in tumor, which were about 2 times that of the CNSI-Fe group. It indicates that the CNSI-Fe after near-infrared irradiation can effectively elevate the temperature in tumor, improve the efficiency of the Fenton reaction and produce more hydroxyl radicals.

### 3) Experiment on inhibiting lymph node metastasis

The inhibitory effects of the CNSI-Fe, CNSI + near-infrared irradiation, CNSI-Fe + near-infrared irradiation, and near-infrared irradiation on the lymph node metastasis of H22 liver cancer cells were tested (FIG. 5). The results showed that compared with the negative group ("*" represented p<0.05, and "**" represented p<0.01), the weight of lymph nodes in the near-infrared irradiation group was not changed obviously; and the weight of lymph nodes in the CNSI-Fe group, the CNSI + near-infrared irradiation group and the CNSI-Fe + near-infrared irradiation group was significantly decreased (p<0.01). The CNSI-Fe + near-infrared irradiation had the greatest inhibitory effect on the lymph node metastasis, the weight of the transferred lymph nodes was the lightest, and there was significant difference (p<0.05) as compared with the CNSI-Fe alone or the CNSI + near-infrared irradiation. According to the calculation using the Jin's formula method, the q value of the CNSI-Fe + near-infrared irradiation was greater than 1, indicating that CNSI-Fe and near-infrared irradiation were synergic and could enhance the inhibitory effects of hyperthermia using CNSI-Fe and CNSI on lymph node metastasis.

It should be stated that the above-mentioned embodiments are only used for explaining, rather than limiting, the technical solutions of the present invention. Although the present invention is explained in detail by reference to the above-mentioned embodiments, those of ordinary skill in the art should understand that modifications or equivalent substitutions may be made to the present invention still, and any modification or local substitution without deviating from the spirit and scope of the present invention should fall into the scope of the claims of the present invention.

## Claims

1. An application of a carbon nanoparticles suspension injection-Fe (CNSI-Fe) compound preparation in preparing a tumor hyperthermia drug to be used in combination with near-infrared light.

2. The application according to claim 1, wherein the "being used in combination with near-infrared light" refers to using near-infrared light to heat a tissue.

3. The application according to claim 1, wherein the near-infrared light has a wavelength of 780-2,600 nm.

4. The application according to any of claims 1-3, wherein the CNSI-Fe compound preparation comprises a carbon nanoparticles suspension injection (CNSI) and ferrous sulfate for injection, and a mass ratio of the CNSI to the ferrous sulfate for injection is 20-100 : 3-300; and preferably, the mass ratio is 50 : 7.5-180.

5. The application according to claim 4, wherein when the CNSI-Fe compound preparation is in use, the CNSI and the ferrous sulfate for injection are uniformly mixed, and a pH value of the mixture is determined to be 2-7; and preferably, the pH value is determined to be 3-5.5.

6. The application according to claim 4, wherein the CNSI comprises carbon nanoparticles, a suspending agent, saline, a pH adjuster, sodium chloride and water for injection; further, each 1,000 mL of CNSI comprises 20-100 g of carbon nanoparticles, 17-30 g of suspending agent, 2-4 g of pH adjuster, 8-10 g of sodium chloride and a remaining amount of water for injection.

7. The application according to claim 6, wherein the carbon nanoparticles are any one or more selected from carbon nanoparticles, carbon nanotubes, carbon quantum dots, graphene, fullerene, carbon nanorods and carbon nanofibers.

8. The application according to claim 6, wherein the suspending agent is any one or more selected from poloxamer, polyvinylpyrrolidone C30 (namely PVPC30) and Tween-80.

9. The application according to claim 6, wherein the pH adjuster is any one selected from sodium citrate, sodium acetate, sodium borate, sodium phosphate and sodium bicarbonate.

10. The application according to claim 4, wherein the ferrous sulfate for injection is a lyophilized powder of ferrous sulfate for injection; and preferably, the lyophilized powder of ferrous sulfate for injection is prepared from the following raw materials: ferrous sulfate heptahydrate, sulfuric acid and water for injection, wherein a mass ratio of ferrous sulfate heptahydrate : sulfuric acid : water for injection is 0.1-0.2 g : 0.4-0.85 µg : 2 g.

11. The application according to claim 10, wherein the ferric salt is selected from any one or more selected from ferrous sulfate, ferric sulfate, ferrous chloride, ferric trichloride, ferrous gluconate, ferric sucrose, ferric ammonium citrate, ferrous succinate, ferric sorbitol and ferrous fumarate.

12. The application according to any of claims 6-9, wherein a preparation method of the CNSI comprises the following steps:
1) taking water for injection in amount of 90% v/v of its formulation amount, adding sodium chloride in its formulation amount, and stirring the mixture until the mixture is completely dissolved to obtain a salt solution;
2) adding pH adjuster and suspending agent in respective formulation amount into the salt solution obtained in step 1), and stirring the mixture until the mixture is completely dissolved to obtain an excipient solution;
3) adding pretreated carbon nanoparticles in corresponding formulation amount into the excipient solution obtained in step 2), stirring the mixture evenly, and then adding the remaining amount of water for injection to a constant volume, so as to obtain a constant-volume solution; and
4) homogenizing the constant-volume solution obtained in step 3) at a rate of 15,000-20,000 rpm for 3-10 min and then at a pressure of 15,000-30,000 psi for 1-5 times, then performing filling and capping, and finally performing steam sterilization at 115-130 °C for 10-30 min to obtain the CNSI.

13. The application according to claim 12, wherein the pretreatment in step 3) comprises the following steps: firstly washing the carbon nanoparticles degreased by ethyl acetate using a 8-15%v/v HNO₃ aqueous solution, wherein a mass-to-volume ratio of the carbon nanoparticles to the HNO₃ aqueous solution is 1 g : 3-5/ml; then washing the carbon nanoparticles with water to be approximately neutral; then washing the carbon nanoparticles with a 0.08-0.15 mol/L NaOH aqueous solution, wherein a mass-to-volume ratio of the carbon nanoparticles to the NaOH aqueous solution is 1 g : 3-5/ml; and washing the carbon nanoparticles with water to be approximately neutral.

14. The application according to any of claims 10-11, wherein a preparation method of the lyophilized powder of ferrous sulfate for injection comprises the following steps:
S1: taking water for injection in amount of 90% of the formulation amount, and continuously filling N₂ below the liquid level during the preparation, with a nitrogen flow rate controlled at 4.0-5.0 m³/h;
S2: then adding sulfuric acid to adjust the pH value to 2.4-2.8;
S3: taking ferrous sulfate heptahydrate in its formulation amount, and fully stirring the ferrous sulfate heptahydrate for dissolution;
S4: adding the remaining amount of sulfuric acid to adjust the pH value to 2.8-3.0, adding the remaining amount of purified water to fix the volume to a full volume, and stirring the mixture evenly to obtain a ferrous sulfate solution; and
S5: filtering the obtained solution using a microporous filter membrane, then performing filling, lyophilization, vacuum plugging and capping, to obtain the lyophilized powder of ferrous sulfate for injection.

15. The application according to claim 14, wherein in S5 of the preparation method of the lyophilized powder of ferrous sulfate for injection, the lyophilization step comprises the following specific operations:
S51 pre-freezing: rapidly lowering the temperature to (-10 to -5) °C and keeping the temperature for 1-3 h, and then rapidly lowering the temperature to (-50 to -40) °C and keeping the temperature for 1-3 h;
S52 drying, comprising
S521: gradually elevating the temperature from (-50 to -40)°C to (-20 to -10)°C within 2-4 h, and keeping the temperature at (-20 to -10)°C and 80-120 mtorr for 2-4 h;
S522: gradually elevating the temperature from (-20 to -10)°C to (-10 to -0)°C within 1-3 h, and keeping the temperature at 80-120 mtorr and (-10 to -0)°C for 1-3 h; and
S523: gradually elevating the temperature from (0-20) °C within 3-6 h, and then keeping the temperature at 80-120 mtorr and 20°C for 3-6 h.
